(19) Europäisches Patentamt
European Patent Office
Office européen des brevets

(11) **EP 1 653 851 B1**

(12) **EUROPÄISCHE PATENTSCHRIFT**

(45) Veröffentlichungstag und Bekanntmachung des Hinweises auf die Patenterteilung:
**12.01.2011 Patentblatt 2011/02**

(51) Int Cl.:
***A61B 5/0402*** (2006.01)   ***G06F 17/00*** (2006.01)

(21) Anmeldenummer: **04762639.5**

(22) Anmeldetag: **06.08.2004**

(86) Internationale Anmeldenummer:
**PCT/DE2004/001794**

(87) Internationale Veröffentlichungsnummer:
**WO 2005/013817 (17.02.2005 Gazette 2005/07)**

(54) **EKG-SYSTEM UND VERFAHREN ZUR GROSSFLÄCHIGEN MESSUNG VON EKG-SIGNALEN**

ECG SYSTEM AND METHOD FOR THE LARGE-SURFACE MEASUREMENT OF ECG SIGNALS

SYSTEME ECG ET PROCEDE DE MESURE A GRANDE SURFACE DE SIGNAUX ECG

(84) Benannte Vertragsstaaten:
**AT BE BG CH CY CZ DE DK EE ES FI FR GB GR HU IE IT LI LU MC NL PL PT RO SE SI SK TR**

(30) Priorität: **07.08.2003 DE 10336809**

(43) Veröffentlichungstag der Anmeldung:
**10.05.2006 Patentblatt 2006/19**

(73) Patentinhaber: **Charité - Universitätsmedizin Berlin**
**10117 Berlin (DE)**

(72) Erfinder:
• **ZABEL, Markus**
  **12161 Berlin (DE)**
• **KOCH, Hans**
  **12159 Berlin (DE)**

(74) Vertreter: **Gross, Felix et al**
**Patentanwälte Maikowski & Ninnemann**
**Postfach 15 09 20**
**10671 Berlin (DE)**

(56) Entgegenhaltungen:
**EP-A- 1 221 299    US-A- 5 377 687**

• **LUX R L ET AL: "CLINICALLY PRACTICAL LEAD SYSTEMS FOR IMPROVED ELECTROCARDIOGRAPHY: COMPARISON WITH PRECORDIAL GRIDS AND CONVENTIONAL LEAD SYSTEMS" CIRCULATION, AMERICAN HEART ASSOCIATION, DALLAS, TX, US, Bd. 59, Nr. 2, 1979, Seiten 356-363, XP008018507 ISSN: 0009-7322**
• **MURRAY A ET AL: "Simplified body-surface electrocardiographic maps with depolarization magnitude and direction" PHYSIOL. MEAS., Bd. 15, 1994, Seiten 235-242, XP002308278**
• **TACCARDI B: "Distribution of heart potentials on the thoracic surface of normal human subjects." CIRCULATION RESEARCH, vol. 12, April 1963 (1963-04), pages 341-352, XP007913365 ISSN: 0009-7330**

**Beschreibung**

Die Erfindung betrifft ein EKG-System mit den Merkmalen des Anspruchs 1 und ein Verfahren zur großflächigen Messung von EKG-Signalen nach Ausspruch 17.

Im klinischen Alltag ist das 12-Kanal EKG der akzeptierte Standard. Insbesondere sind die Elektrodenpositionen am Körper genau festgelegt. Auch ist festgelegt, in welcher Weise die EKG-Signale abgeleitet, verrechnet und grafisch dargestellt werden. Eine detaillierte Darstellung ist aus "Comprehensive Elektrocardiography - Theory and Practice in Health and Desease", Band 1, Herausgeber P.W.Macfarlane und T.D. Veitch Lawrie, Pergamon Press, New York, 1989, insbesondere Kapitel 11 "Lead Systems" bekannt.

Mit dem klassischen 12-Kanal-EKG Verfahren wird an sechs Brustwandpositionen ($V_1$ bis $V_6$) das elektrische Potential gemessen. Hinzu kommen sechs Extremitätenableitungen (I, II, III, aVL, aVR, aVF). Dabei wurde bereits als nachteilig erkannt, dass die von der Herztätigkeit generierten elektrischen Potentialänderungen großflächig über den Körper ausgebreitet sind. Bei bestimmten Krankheitsbildern liegen charakteristische Veränderungen in Thoraxbereichen vor, die von den klassischen EKG-Elektroden nicht erfasst werden.

Es ist daher aus klinischer Sicht wünschenswert, über einen größeren Thoraxbereich EKG-Signale abzuleiten.

Dies wird z.B. durch das sogenannte Body-Surface-Potential-Mapping (BSPM) erreicht, wobei nachgewiesen wurde, dass damit zusätzliche klinisch relevante Daten ermittelbar sind (N.C. Flower, L.G. Horan in "Body Surface Potential Mapping", Kapitel 82 in "Cardiac Electrophysiology - From Cell to Bedside", 3. Auflage, Herausgeber D.P. Zipes und J. Jalife, W.B. Saunders, Philadelphia, 2000).

Bisher konnte sich dieses Verfahren, bei dem an 20 bis 200 Elektrodenpositionen simultan das elektrische Potential gemessen wird, auf Grund seiner Komplexität und der damit verbundenen hohen Kosten im Klinikalltag nicht durchsetzen. Es ist bekannt, die klassischen 12-Kanal-EKG Messungen mit zusätzlichen Messungen an anderen Elektrodenpositionen zu ergänzen, um die klinische Diagnose zu verbessern (z.B. A.P. Michaelides et al. "Improved detection of coronary artery disease by exercise electrocardiography with the use of right precordial leads" N. Eng. J. Med. 340 (1999) 5).

Allerdings wird bei diesen Untersuchungen kein Verfahren benutzt, das eine ausreichend genaue Synchronisation der Einzelsignale ermöglicht, um ein "Mapping" im Sinne des BSPM zu erreichen.

Aus dem Artikel von Bruno Taccardi, "Distribution of Heart Potentials on the Thoracic Surface of Normal Human Subjects", (Circulation Research, Volume XII, April 1963) ist weiterhin ein Verfahren bekannt, bei dem diverse Thoraxableitungen in Verbindung mit einem ebenfalls am Thorax abgeleiteten Referenz-EKG zur Bestimmung des Herzpotentials an der Brustwand eines gesunden Probanden verwendet werden. Hierbei muss allerdings durch einen Rechteckwellengenerator nach jeder Herzkontraktion ein Kalibrierungssignal erzeugt werden, was den Nachteil aufweist, dass ein entsprechender apparativer Aufwand betrieben werden muss.

Der vorliegenden Erfindung liegt daher die Aufgabe zugrunde, ein Verfahren zur großflächigen Aufnahme von EKG Signalen zu entwickeln, das aber leicht und effizient im Klinikalltag anwendbar ist. Ein EKG-System, das die Merkmale des Oberbegriffs der Anspruchs 1 umfasst, ist aus der US-A-5 377 687 bekannt.

Das erfindungsgemäße EKG-System verwendet ein erstes Messmittel zur Erzeugung eines ersten Messdatensatzes enthaltend mindestens eine Ableitung der Herzströme, wobei mindestens ein Ableitungsort des ersten Messmittels (10) während der Aufnahme der großflächigen EKG-Signale variabel ist. Ferner wird zeitgleich ein zweites Messmittel verwendet zur Erzeugung eines zweiten Messdatensatzes enthaltend mindestens eine Ableitung der Herzströme, wobei der Ableitungsort des zweiten Messmittels während der Aufnahme der großflächigen EKG-Signale zum Erhalten kontinuierlicher Messergebnisse räumlich unveränderlich ist. Schließlich weist das erfindungsgemäße EKG-System ein Datenverarbeitungssystem mit einem Mittel zur Synchronisation mindestens zweier zeitlich versetzt ermittelter Signale des ersten Messdatensatzes mit mindestens einem kontinuierlich ermittelten Signal des zweiten Messdatensatzes auf. Damit ist es möglich, mindestens ein diskontinuierlich gewonnenes Signal des ersten Messdatensatzes durch mindestens ein kontinuierlich gewonnenes Signal des zweiten Messdatensatzes zu synchronisieren. Ein solches System ist z.B. in der Intensivmedizin einsetzbar. Die räumliche Versetzung der Messstellen ist am liegenden Patienten in einfacher Weise möglich.

Dabei ist es vorteilhaft, wenn der erste Messdatensatz Messungen von Herzströme enthält, die an Thorax-Ableitungen ($V_1$ - $V_6$) gewonnen sind. Besonders vorteilhaft ist es, wenn der erste Messdatensatz Messungen der Herzströme aus einer zeitlichen Folge von Thorax-Ableitungen ($V_1$ - $V_6$) an unterschiedlichen Thoraxpositionen enthält. Damit kann dann eine großflächige Gewinnung der EKG-Daten erfolgen.

Eine vorteilhafte Möglichkeit der Gewinnung von kontinuierlichen Messdaten ist, wenn der zweite Messdatensatz mindestens eine Messung der Herzströme einer Extremitätenableitung (I, II, III, aVR, aVL, aVF) enthält. Besonders vorteilhaft ist es, wenn der zweite Messdatensatz Signale der Herzströme aller Extremitätenableitungen (I, II, III, aVR, aVL, aVF) enthält.

In einer vorteilhaften Ausgestaltung des erfindungsgemäßen EKG-Systems erfolgt die Synchronisation anhand mindestens eines markanten Signalmusters des zweiten Messdatensatzes.

Dabei ist es vorteilhaft, wenn das Mittel zur Synchronisation das Signal einer R-Zacke im zweiten Messdatensatz zur Synchronisation verwendet. Besonders vorteilhaft ist es, wenn das Mittel zur Synchronisation das Signal der Steigung der R-Zacke im zweiten Messdatensatz zur Synchronisation verwendet.

Ferner ist es vorteilhaft, wenn das Mittel zur Synchronisierung markante Signalmarker mehrerer gemessener EKG-Kanäle verwendet.

Eine weitere vorteilhafte Ausgestaltung des erfindungsgemäßen EKG-Systems weist einen Filter, ein Mittel zur Mittelung und /oder zur Ermittlung des Medians für Signale des ersten Messdatensatzes und / oder des zweiten Messdatensatzes auf. Damit lassen sich charakteristische Herzschläge ermitteln, die für die Synchronisation verwendet werden.

Auch ist es vorteilhaft, wenn das EKG-System ein Mittel zur Korrektur der Basisline individueller Herzströme aufweist.

Mit Vorteil weist eine Ausführungsform des erfindungsgemäßen EKG-Systems ein Datenverarbeitungssystem auf, das für jeden beliebigen Zeitpunkt einer Messung relativ zu einer mittels eines Signals des zweiten Messdatensatzes gewonnenen Zeitreferenz aus den Amplitudenwerten aller Thorax-Ableitungen automatisch eine graphische Darstellung der momentanen Potentialverteilung ermittelt.

Dabei ist es vorteilhaft, wenn die graphische Darstellung eine QRST-Integral-Map Darstellung ist.

Für die Aufnahme der großflächigen EKG-Signale ist es vorteilhaft, wenn das erstes Messmittel und / oder das zweite Messmittel in einer am menschlichen Körper tragbaren Vorrichtung, insbesondere einer Weste angeordnet sind. Damit ist z.B. eine Langzeit-EKG-Untersuchung möglich.

Für die Prüfung der Wirksamkeit einer Messung ist es vorteilhaft, wenn mittels des Datenverarbeitungssystems eine Varianz von Messergebnissen als Validitätskenngröße ermittelbar ist. Besonders vorteilhaft ist es dabei, wenn die Varianz der Messergebnisse anhand eines Maßes bestimmter EKG-Potentialhöhen, insbesondere von RR-Abständen, QT-Zeiten und / oder eines Vergleiches eines Mittelwertes eines Maßes für eine EKG-Potentialhöhe einer Messphase mit dem Mittelwert für Maße von EKG-Potentialhöhen aller Messphasen ermittelbar ist.

Die Aufgabe wird auch durch ein Verfahren zur großflächigen Aufnahme von EKG-Signalen mit den Merkmalen des Anspruchs 17 gelöst.

Durch eine Aufnahme mindestens einer ersten Messung der Herzströme mit einem ersten Messmittel, wobei mindestens ein Ableitungsort eines ersten Messmittels während der Aufnahme der großflächigen EKG-Signale verändert wird, und eine zeitgleich erfolgende Aufnahme mindestens einer zweiten Messung der Herzströme mit einem zweiten Messmittel, wobei der Ableitungsort des zweiten Messmittels während der Aufnahme der großflächigen EKG-Signale zur kontinuierlichen Messung räumlich unveränderlich ist, werden zwei Datensätze erzeugt, die effizient zu einander in Beziehung gesetzt werden können.

Sofort oder zu einem späteren Zeitpunkt werden dann mindestens zwei zeitlich versetzt ermittelte Signale der Herzströme des ersten Messdatensatzes mit mindestens einem kontinuierlich ermittelten Signal des zweiten Messdatensatzes der Herzströme in einem Datenverarbeitungssystem automatisch synchronisiert.

Dabei ist es vorteilhaft, wenn insbesondere zur Simulation eines Body-Surface-Potential-Mappings mindestens zwei erste Ableitungen durch einen Interkostalabstand getrennt am Thorax gewonnen werden.

Im Folgenden wird anhand von Ausführungsbeispielen das erfindungsgemäße System beschrieben, mit dem es möglich ist, durch Verwendung allgemein verfügbarer digitaler 12-Kanal-EKG-Systeme Näherungen von Body Surface Potential Mappings (BSPM) herzustellen. Diese Pseudo-BSPMs enthalten den größten Teil der durchschnittlichen räumlich-zeitlichen Information eines einzigen charakteristischen Herzschlags. Die zugrundeliegende Signalverarbeitung wird im Detail beschrieben. Die Algorithmen können auf einfache Weise der Software von kommerziellen 12-Kanal-EKG-Geräten zugefügt werden.

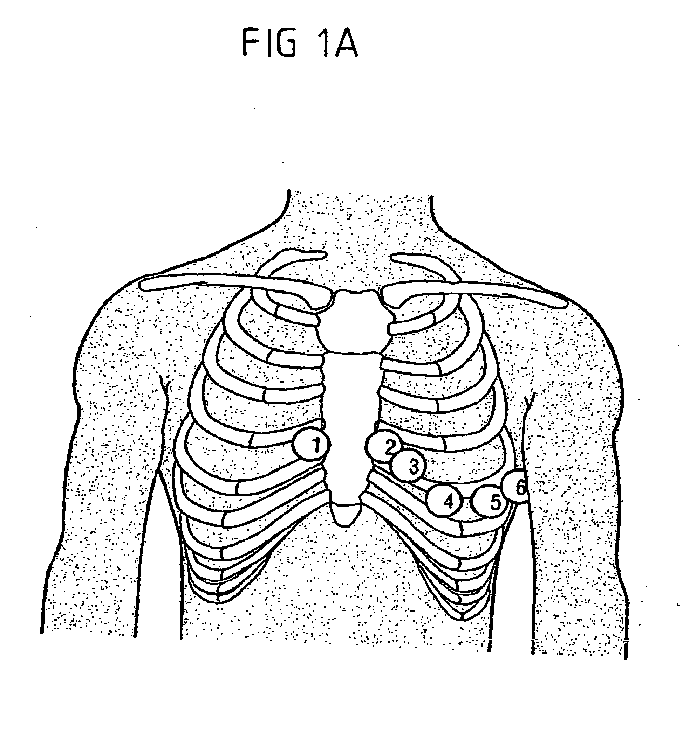

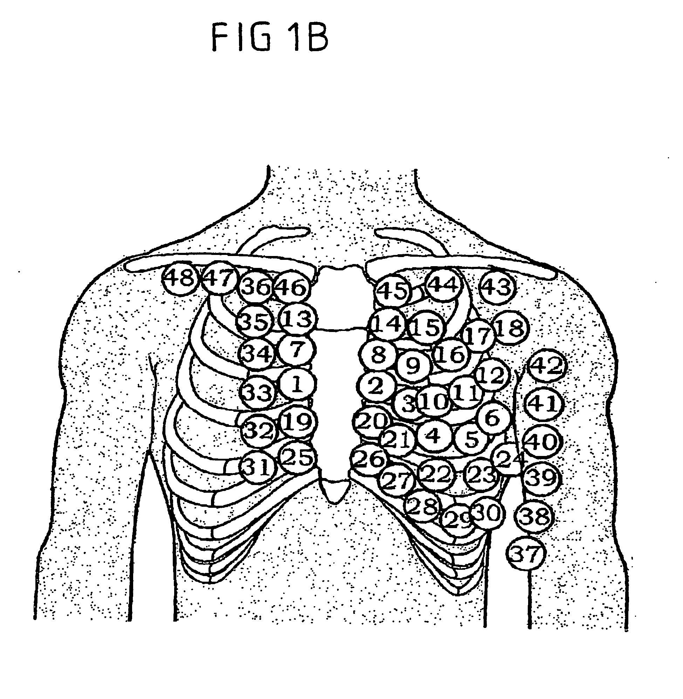

Body-Surface-Potential-Mapping (BSPM) ist nachweislich eine klinisch relevante Methode, die die diagnostische Leistung verglichen zum Standard-12-Kanal-EKG erhöht. Eine Übersicht geben (siehe N.C. Flowers, L.G. Horan: "Body Surface Potential Mapping" in D.P. Zipes, J. Jalife (eds): "Cardiac Electrophysiology: From Cell to Bedside", 2nd ed., Philadelphia, WB Saunders, 1995, pp 1049-1067; N.C. Flower, L.G. Horan in "Body Surface Potential Mapping", Kapitel 82 in "Cardiac Electrophysiology - From Cell to Bedside", 3. Auflage, Herausgeber D.P. Zipes und J. Jalife, W.B. Saunders, Philadelphia, 2000; "Comprehensive Electrocardiology-Theory and Practice in Health and Disease", vol. 1; "Comprehensive Elektrocardiography - Theory and Practice in Health and Desease", Band 1, Herausgeber P.W.Macfarlane und T.D. Veitch Lawrie, Pergamon Press, New York, 1989, insbesondere Kapitel 11 "Lead Systems") und die darin enthaltenen Quellenangaben. Der Grund für eine solche verbesserte Detektion und Separation von pathophysiologischen Herzfunktionen durch BSPM liegt begründet in der wesentlich größeren Anzahl von Messpositionen der Elektroden, die am Thorax befestigt sind. Figuren 1a und 1b geben einen sofortigen Eindruck über die verbesserte Abdeckung des Thorax und der dadurch verbesserten Detektion von wichtigen räumlichen Komponenten der elektrischen Potentialverteilung durch BSPM (Figur 1b), die sonst den sechs Standard-Brustelektroden der 12-Kanal-EKG (Figur 1a) entgangen wären. Zu unterschiedlichen Zeitpunkten ist die Potentialverteilung häufig räumlich erheblich verändert. Daher können räumlich und zeitlich wichtige Eigenschaften nicht durch das 12-Kanal-EKG eingefangen werden, mit Hilfe des BSPM ist dies aber möglich.

An dieser Stelle sollte erwähnt werden, dass Figur 1b nicht die übliche Verteilung der BSPM-Elektroden darstellt, sondern die Konfiguration, die für die hier vorgestellte Methode ausgewählt wurde. Für herkömmliches BSPM ist die Anzahl und die Verteilung der Elektroden auf dem Thorax nicht genauso standardisiert wie im Fall der sechs Brustelektroden-Positionen des 12-Kanal-EKGs. Obwohl nur wenige Konzepte der Elektroden-Konfiguration etabliert wurden, ist keine davon tatsächlich weltweit akzeptiert. Es könnte dahingehend argumentiert werden, dass für BSPM eine exakte Positionierung der Elektroden nicht so wichtig ist, wie für die $V_1$-$V_6$-Elektroden des 12-Kanal-EKGs, da aufgrund des Mappings alle relevanten Eigenschaften irgendwie eingefangen werden - wenn nicht durch eine bestimmte Elektrode, dann durch eine der benachbarten Elektroden.

Bezüglich der optimalen Anzahl der Elektroden für BSPM gibt es einen Kompromiss zwischen der Komplexität (und somit den Kosten) und der Detektion von relevanter Information. Komplexität und Kosten sind das Haupthindernis, das einen Durchbruch des BSPM in der klinischen Routine verhindert hat. Das aber ist die Motivation dieser Erfindung: Ein System und eine Methode zu schaffen, die vergleichbare - wenn auch nicht identische - Ergebnisse erzielt wie BSPM aber nur eine Standard-12-Kanal-EKG-Instrumentierung benötigt.

In Abgrenzung zum Voll-BSPM wird die Methode Pseudo-BSPM genannt. Der Hauptunterschied zum echten BSPM ist, dass nicht alle Kanäle gleichzeitig ausgelesen werden, d.h. das Mapping wird aus sequenziell erhaltenen EKG-Signalen rekonstruiert. Auf der anderen Seite stammen die meisten in der BSPM-Literatur als klinisch relevant bezeichneten Eigenschaften von gemittelten Daten und enthalten keine Information über eine Variabilität von Herzschlag zu Herzschlag. Daher ist der Unterschied zwischen den graphischen Darstellungen, die durch echtes BSPM und durch das hier vorgestellte Pseudo-BSPM gewonnen werden, nicht wesentlich.

Die vorliegende Erfindung soll anhand eines Ausführungsbeispiels beschrieben werden, wie die sequentiell aufgezeichneten EKG-Signale eines kommerziellen 12-Kanal-EKG-Systems auf eine Weise synchronisiert werden, dass es möglich ist, gültige Näherungen von BSPMs zusammenzustellen.

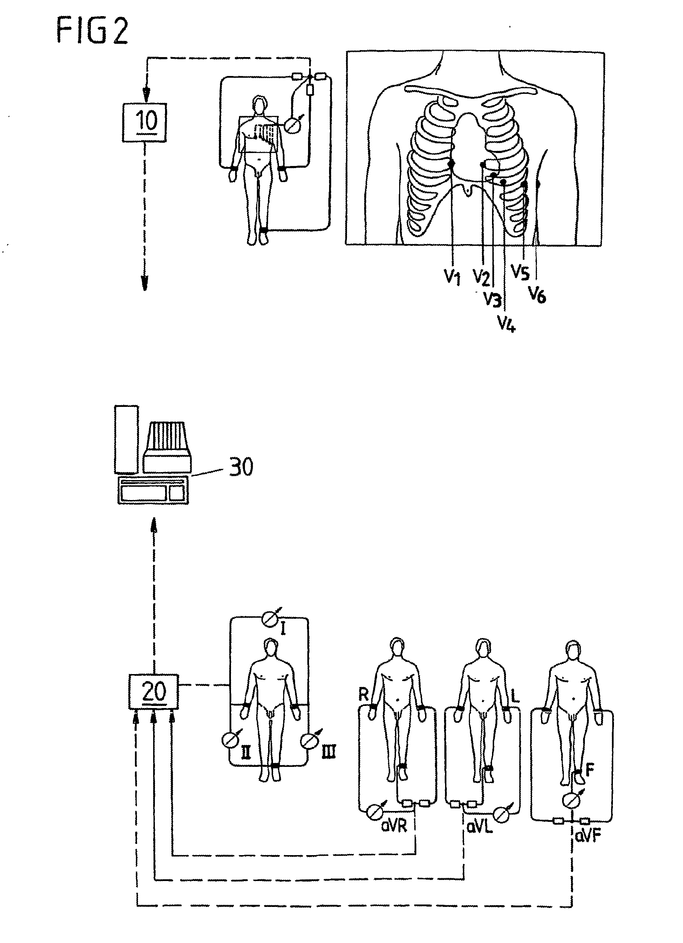

In Figur 2a sind gebräuchliche Ableitungsformen eines 12-Kanal EKGs beschrieben. Im oberen Teil der Figur 2 sind die Thoraxableitungen ($V_1$ bis $V_6$) dargestellt. Die bipolaren Extremitätenableitungen (I, II, III) und die unipolaren Extremitätenableitungen (aVR, aVL, aVF) sind im unteren Teil dargestellt.

Das erfindungsgemäße System weist ein hier schematisch dargestelltes erstes Messmittel 10 auf, das die Signale der Thoraxableitungen misst. Mindestens eine räumliche Lage der Thoraxableitungen wird aber im Laufe einer vollständigen Messung sukzessive verändert. Somit wird innerhalb einer vollständigen Messungen mindestens eine Ableitung $V_1$ bis $V_6$ an unterschiedlichen Stellen ermittelt. Im Folgenden soll angenommen werden, dass alle Thoraxableitungen nach dem Ende eines ersten Messabschnittes räumlich am Thorax versetzt werden. Dies kann z.B. jeweils ein Interkostalabstand sein.

Demgegenüber bleiben die Extremitätenableitungen, die von einem schematisch dargestellten zweiten Messmittel 20 aufgenommen werden, während einer vollständigen Messung räumlich unverändert. Wie später noch ausführlich dargelegt wird, dienen diese Ableitungen der Erzeugung einer Pseudo-Synchronisation der räumlich an unterschiedlichen Orten abgenommenen Thoraxableitungen.

Die Extremitätenableitungen werden in einer Ausführungsform der Erfindung auch dazu verwendet, mindestens eine Validitätskenngröße zu ermitteln. Die Validitätskenngröße ist ein Maß dafür, ob die Grundvoraussetzung für das erfindungsgemäße Verfahren während der gesamten Messdauer erfüllt ist, nämlich die weitgehende Konstanz des Herzschlagmusters. Dies wird unten noch näher erläutert.

Die Pseudo-Synchronisation wird durch ein Datenverarbeitungssystem 30 durchgeführt, das als Hardware oder Software implementiert, ein Mittel aufweist, mit dem die Messsignale des ersten Messmittels 10 und des zweiten Messmittels 20 automatisch miteinander synchronisierbar sind.

Die folgende Beschreibung fokussiert nur auf einen Prozess, mit dem man durch Verwendung eines kommerziellen digitalen 12-Kanal-EKG-Systems ein Pseudo-BSPM erhält.

**Datenproduktion**

Die Datenproduktion für das Pseudo-BSPM kann mit jedem Standard-12-Kanal-EKG-Gerät durchgeführt werden, soweit ein digitaler Datenausgang eine Wiederherstellung von digitalisierten Signalen für eine numerische Offline-Rekonstruktion erlaubt.

Eine typische Aufzeichnungs-Session beinhaltet folgende verschiedene Phasen:

Phase 1:

Zuerst wird eine Standard-12-Kanal-EKG-Aufzeichnungsprozedur begonnen, d.h., es werden die Elektroden an den standardisierten Positionen des Thorax ($V_1$-$V_6$) (vgl. Figur 1a, 2) und der Extremitäten (I, II, III, aVR, aVL, aVF) angebracht, und die 12-Kanal-EKG-Signale werden mit den Messmitteln 10, 20 aufgezeichnet.

Phase 2:

Nun werden die Brustelektroden ($V_1$-$V_6$) um einen Rippenabstand nach oben verschoben, während die Elektrodenpositionen der Extremitäten unverändert bleiben. Dann werden die 12-Kanal-EKG-Signale der Phase 2 mit dem Messmitteln 10, 20 aufgezeichnet. Generell sollten die Signalaufzeichnungen schnell hintereinander folgen, wobei genau darauf geachtet werden sollte, Rhythmusstörungen oder Veränderungen der basalen Herzrate zu verhindern.

Phase 3 bis 8:

Diese Phasen werden in gleicher Weise durchgeführt, wodurch eine sequentielle Abdeckung der Thoraxelektroden-Positionen resultiert, wie in Figur 1b dargestellt.

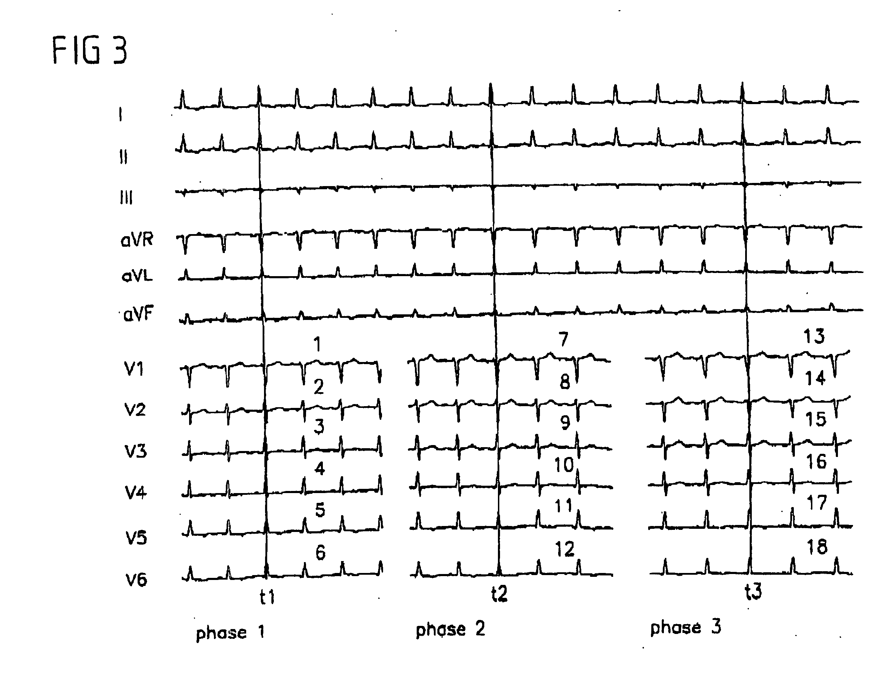

Zum Schluss sollten die im Datenverarbeitungssystem 30 gespeicherten EKG-Signale, ein Aufzeichnungsformat haben, wie es schematisch für die ersten drei Phasen der Figur 3 gezeigt ist. Es sollte beachtet werden, dass die Aufzeichnung in Phase 1 mit einem vollständigen standardisierten 12-Kanal-EKG beginnt. Somit geht keine Information verloren, verglichen mit dem Standard-EKG, alles, was als nächstes folgt ist zusätzliche (oder redundante) Information.

In Phase 2 enthalten die EKG-Spuren, die normalerweise für $V_1$-$V_6$ reserviert sind, nun die aufgezeichneten Signale der Positionen 7 bis 12, während die Spuren I, II, III, aVR, aVL, aVF kontinuierliche Aufzeichnungen des zweiten Messmittels 20 der in ihrer Lage unveränderten Extremitätenelektroden sind.

**Pseudo-Synchronisation**

Das Ziel von BSPM ist es, für einen bestimmten Zeitpunkt - z.B. dem Peak des QRS-Komplex in Kanal $V_1$ - mittels des Datenverarbeitungssystems 30 eine graphische Darstellung der räumlichen Verteilung des elektrischen Potentials der Thoraxoberfläche zu erhalten. Die normale Prozedur mit einem Standard-BSPM-System funktioniert so, dass von den EKG-Signalen aller Kanäle die momentane Signal-Amplitude bezogen auf diesen Zeitpunkt gesammelt wird, und mit allgemein zugänglichen Algorithmen eine Oberflächen-Anpassung mit Gitterpunkten bezogen auf die Elektrodenpositionen zusammengestellt wird. Diese Oberflächen-Anpassungs-Funktion wird dann entweder als ein Kontur-Plot, eine Grauskala oder ein Falschfarben-Plot dargestellt. Da alle BSPM-Kanäle echt simultan arbeiten, ist es automatisch gewährleistet, dass alle Datenpunkte, die die graphische Darstellung ergeben zum selben Zeitpunkt gemessen wurden.

Mit der Pseudo-BSPM-Methode wie sie hier vorgestellt wird, ist die Situation völlig anders, da die Datenpunkte in den zu konstruierenden graphischen Darstellungen von äußerst unterschiedlichen Zeitpunkten stammen. Wie in Figur 3 gezeigt, müsste man für die Elektrodenpositionen 1 bis 6, gemessen zum Zeitpunkt $t_1$, von den Positionen 7 bis 12, gemessen zum Zeitpunkt $t_2$, von den Positionen 13 bis 18, gemessen zum Zeitpunkt $t_3$, usw. Datenpunkte sammeln, um eine graphische Darstellung zusammenzustellen.

Somit muss eine einzige graphische Darstellung von mindestens acht verschiedenen EKG-Herzschlägen konstruiert werden, die mit einigen Minuten Zeitunterschied gemessen wurden.

Natürlich ist eine solche graphische Darstellung keinesfalls repräsentativ für einen bestimmten Zeitpunkt. Dennoch ist es möglich eine graphische Darstellung zu konstruieren, die eine gute Annäherung eines echten BSPM darstellt, wenn die einzelnen EKG-Herzschlag-Signale von allen acht Phasen in einer angemessenen Weise "übereinandergelegt" werden, um einen "charakteristischen Herzschlag" zu bilden. Wenn entschieden wird, dass - selbst für leicht variierende Pulsraten - Eigenschaften des EKG-Herzschlag-Musters aufrechterhalten bleiben, dann kann eine solche Konstruktion immer noch klinisch relevante Information enthalten. Was verloren geht, ist die Variabilität von Herzschlag zu Herzschlag.

Es sollte hier betont werden, dass es essentiell ist, die Bezugszeitpunkte $t_1$ bis $t_8$ (vgl. Figur 3) so präzise wie möglich festzulegen. Um eine bestmögliche Annäherung zum echten BSPM zu bekommen, muss Wert darauf gelegt werden, die "richtigen" Referenzzeiten zu finden. Dieses Problem soll durch folgendes Beispiel näher erläutert werden:

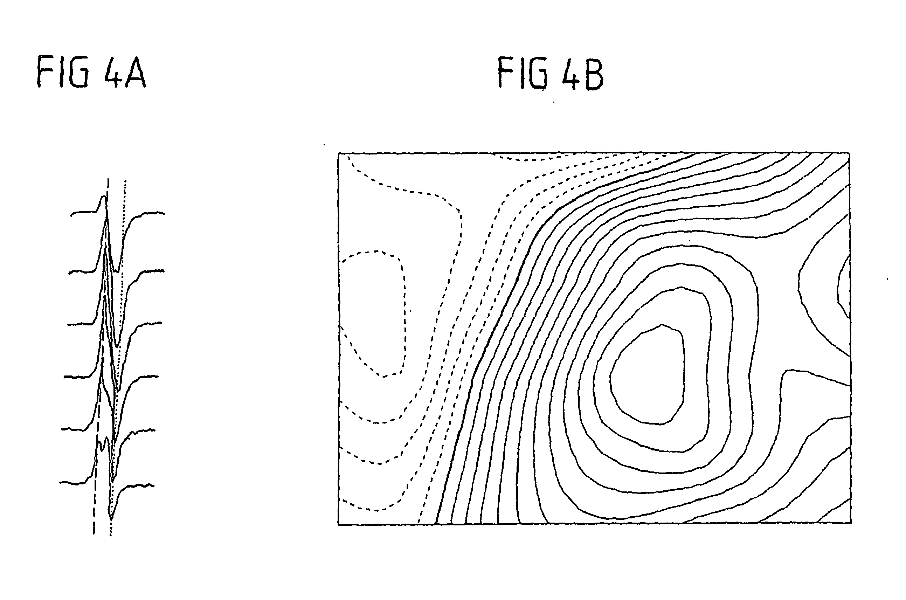

In Figur 4a ist eine Sammlung von gleichzeitig aufgezeichneten EKG-Signalen des QRS von ausgesuchten Elektrodenpositionen gezeigt. Es ist offensichtlich, dass die Extrema der individuellen Signale nicht während derselben Zeit auftauchen. Figur 4b zeigt das entsprechende BSPM für den Zeitpunkt, der in Figur 4a als gestrichelte Linie dargestellt ist.

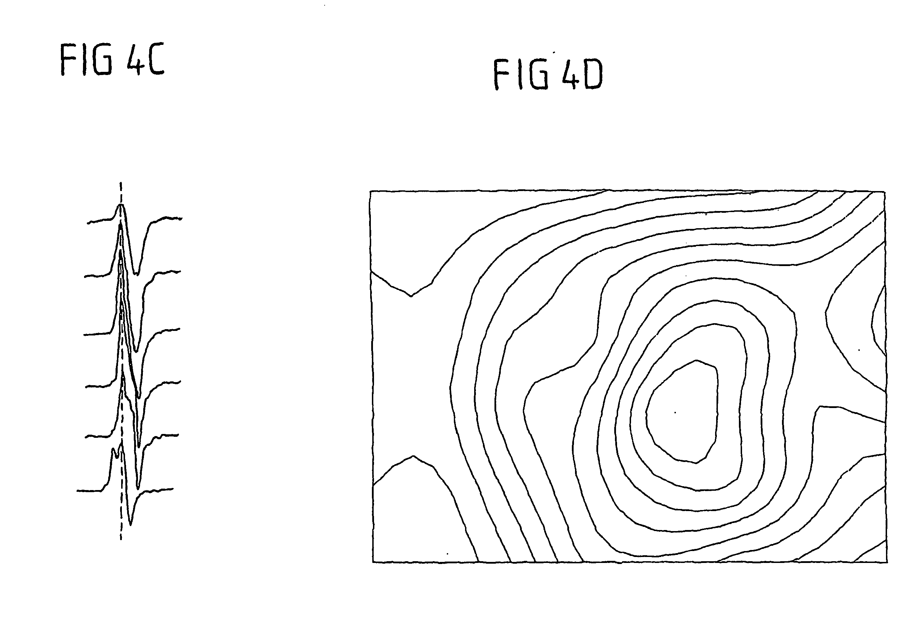

Normalerweise wird für ein BSPM keine Aufnahme der Extremitätenelektroden durchgeführt. Wenn, wie bei dem erfindungsgemäßen System nur die 48 Brustelektroden in sequentieller Weise wie oben beschrieben aufgezeichnet worden wären, dann wäre es schwierig zu entscheiden, wie man die individuellen EKG-Herzschlag-Muster zeitlich einander zuordnen sollte, um sie "korrekt" zeitlich übereinander zu legen, d.h. um ein ähnliches Bild zu bekommen wie in Figuren 4a und 4b dargestellt. Wegen der sequentiellen Aufzeichnung geht die realistische Zeitinformation verloren. Offensichtlich würde eine Ausrichtung an den Peak-Eigenschaften zu einem falschen Ergebnis führen, wie in Figur 4c und der dazugehörigen graphischen Darstellung in Figur 4d gezeigt.

Ausgehend von Figur 4a wird suggeriert, den Anfang der Q-Zacke als übereinstimmenden Zeitpunkt auszuwählen. Dies würde in der Tat in diesem Beispiel funktionieren. Dennoch ist es bekannt, dass dieser Anfang in vielen anderen Fällen von Kanal zu Kanal erheblich variieren kann und häufig nicht besonders gut ausgeprägt ist. Wenn man einen Kanal/Elektrode während der gesamten sequentiellen Aufzeichnung in einer Position behalten würde, sagen wir $V_1$, und man beispielsweise den R-Peak als Trigger benutzen würde, um alle anderen Herzschlag-Signale zu synchronisieren, dann würde dies in vielen Fällen nicht zuverlässig funktionieren. Auch ist der Beginn des QRS-Komplexes eines Kanals häufig nicht ausgeprägt genug und produziert uneindeutige Ergebnisse.

Eine verlässlichere Methode, die zu besseren Ergebnissen führt wird dagegen im Folgenden beschrieben: Die Verlässlichkeit, um adäquate Referenzzeitpunkte zu erhalten, wird erhöht, wenn der Zeitmarker von mehreren Kanälen abgeleitet wird. Sehr ausgeprägte Eigenschaften können erlangt werden, wenn die Summe der Quadrate der Veränderungsrate der Signalamplitude a(t) über alle Extremitätenelektroden für jeden Zeitschritt $t_i$ berechnet wird:

$$r(t_i) = \sum_{n=1}^{6} [a_n(t_i - \Delta t) - a_n(t_i + \Delta t)]^2 \qquad (1)$$

Diese Messung steht in enger Verbindung zu der Varianz der Steigungen dieser Kanäle.

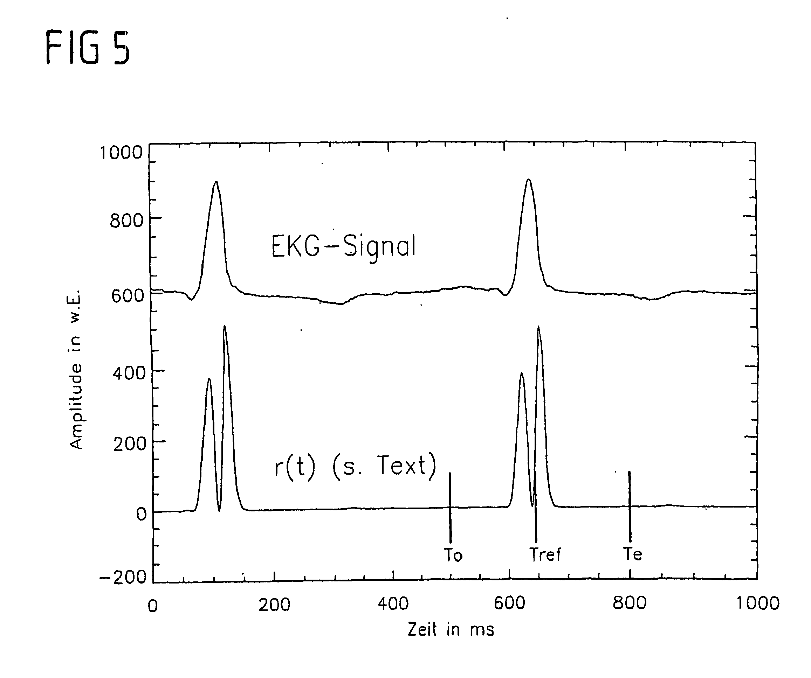

Figur 5 zeigt anhand der unteren Kurve die bessere Unterscheidung zwischen QRS-Eigenschaften und anderen Signalkomponenten wie die T-Welle, wenn man r(t) mit einer EKG-Spur vergleicht. Die obere Kurve ist der Kanal I, der zu Vergleichszwecken abgebildet ist.

Allerdings kann dieses neue Signal nicht ausreichend als verlässlicher Marker angesehen werden, da es möglich ist, dass anstelle von einem Peak zwei oder mehr Peaks vorkommen, die dann eine Mehrdeutigkeit bei der Determinierung des Referenzzeitpunkts hervorrufen. Um eindeutig zu werden, ist es notwendig eine universelle Zeit zu bestimmen, was erreicht wird, wenn der "zeitliche Schwerpunkt" dieser "abgeleiteten" QRS-Eigenschaft wie folgt berechnet wird:

$$T_{ref} = T_0 + \frac{\sum_{t_i=T_0}^{Te} r(t_i) t_i}{\sum_{t_i=T_0}^{Te} r(t_i)} \qquad (2)$$

In r(t) hat die QRS-Eigenschaft ein ausgeprägt gutes Signalrauschverhältnis. Nur die starken Amplituden tragen wesentlich zu den Summen in Gleichung (2) bei und daher ist eine sehr stabile Determination eines realistischen Referenzzeitpunkts möglich, der fast unbeeinflusst ist durch die Wahl der Größe des Zeitfensters.

## Zusammensetzung eines charakteristischen Herzschlags

Sind die Referenzzeitpunkte, wie oben beschrieben, einmal determiniert, kann der Rest der Ausrichtungs-Prozedur in einfacher Weise durchgeführt werden: die individuellen Herzschlagsignale der Kanäle $V_1$ bis $V_6$ aller Phasen müssen entsprechend übereinandergelegt werden.

In vielen Fällen haben realistische klinische EKG-Daten ein erhebliches Rauschen und würden in einem verzerrten, ungleichmäßigen/gezackten BSPM resultieren. Um realistisch geglättete Ergebnisse zu erhalten wird daher vorgeschlagen, geeignet gemittelte Signale zu bilden. In BSPM-Diagnosen werden normalerweise nur die Eigenschaften gewertet, die über einzelne Herzschläge gemittelt sind. Wie bei dieser Methode geeignet gemitteltes Singale (im Folgenden "charakteristisches Herzschlagsignal" genannt) gebildet werden können wird im Folgenden beschrieben:

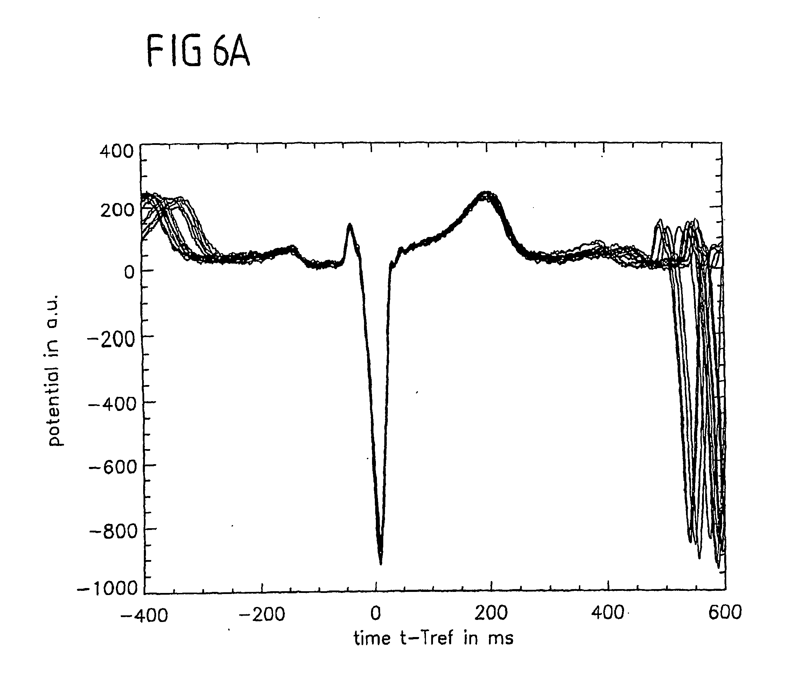

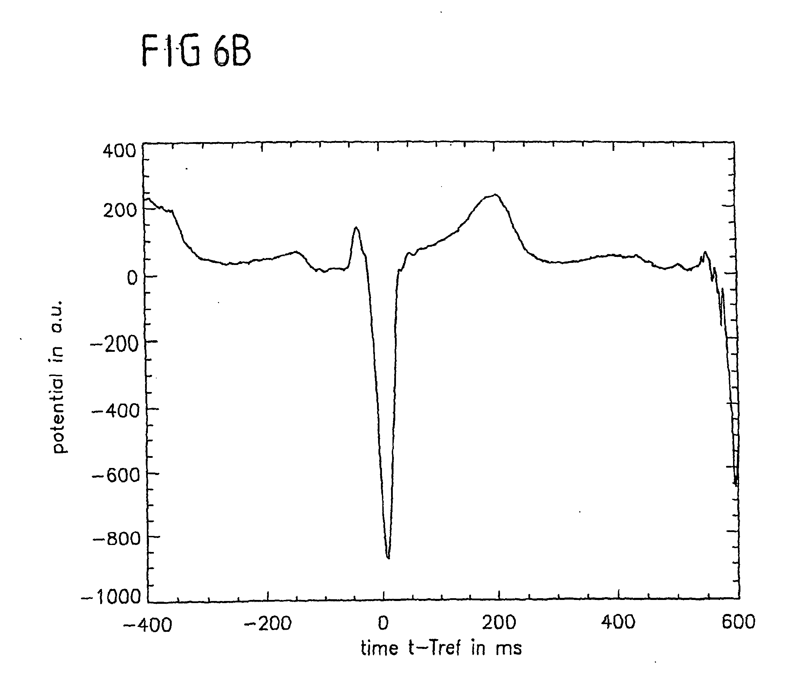

Figur 6 zeigt, wie man ein gemitteltes Signal für einen Kanal und eine Phase erhält, d.h. das charakteristische HerzschlagSignal für eine Elektroden-Position. Die Referenzzeitpunkte für jeden Herzschlag werden wie oben beschrieben bestimmt, und die individuellen Schläge werden an dieser Referenzzeit zeitlich ausgerichtet und übereinandergelegt. Zusätzlich ist es dienlich, die Basis-Linien der individuellen Herzschlag-Signale vertikal anzupassen. Auf diese Weise entsteht Figur 6a.

Die Veränderungen von Herzschlag zu Herzschlag sind im Vergleich der individuellen EKG-Muster deutlich erkennbar. Innerhalb des Zeitintervalls vom Beginn der P-Welle bis zum Ende der T-Welle sind diese Veränderungen von Herzschlag zu Herzschlag nicht besonders ausgeprägt und es kann von einem Gleichgewicht zwischen Depolarisation und Repolarisaton ausgegangen werden. Diese Tatsache ist eine Rechtfertigung des erfindungsgemäßen Systems.

Weiterhin kann aus der Variation der vorausgehenden T-Welle und der folgenden QRS ersehen werden, dass schon bei diesen wenigen Herzschlägen (in der Größenordnung von 10) einer Phase (10 Sekunden) eine nennenswerte Variation von Pulsraten auftaucht. Von der zweifachen Akkumulation der vorausgehenden T-Wellen-Signale kann man sogar auf einen T-Wellen- oder rate-alternans für diesen individuellen Patienten schließen. Schließlich kann das charakteristische Herzschlagsignal (entsprechend Fig. 6b) dieser Elektroden-Position extrahiert werden durch Bestimmung des Medians aller individueller Signalamplituden zu jedem Zeitschritt. Man könnte ebenso den Mittelwert nehmen, aber der Median ignoriert artefaktische oder extra-systolische Herzschlagsignale effektiver und sollte daher bevorzugt werden. In Figur 6b ist das erhaltene abgeleitete charakteristische Herzschlagsignal für eine Elektroden-Position gezeigt.

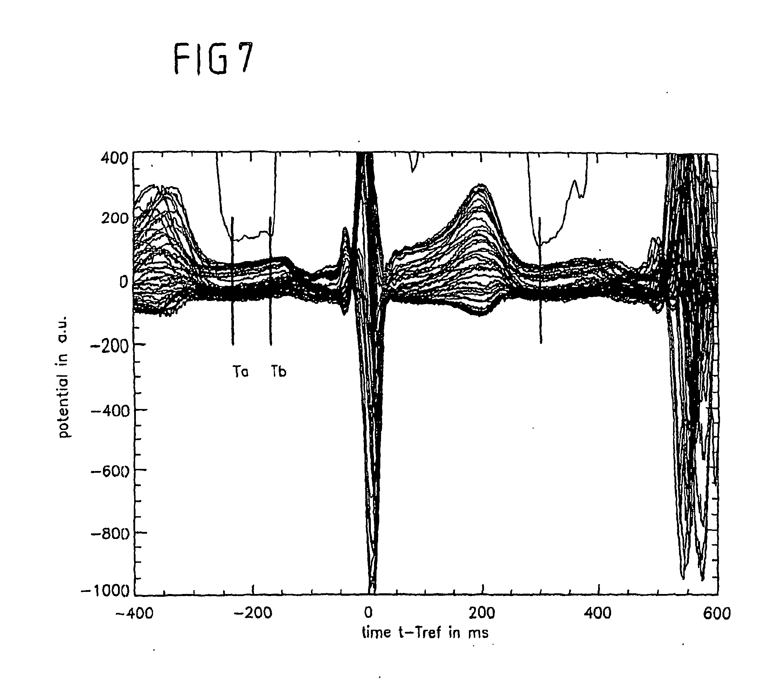

Wenn die charakteristischen Herzschlagsignale für alle Elektroden-Positionen, wie sie in Figur 2b dargestellt sind, übereinandergelegt sind, ergibt sich Figur 7. Die Datensituation dieses Verarbeitungsstadiums kann aber noch nicht genutzt werden, um ein Pseudo-BSPM zu erzeugen. Die resultierende graphische Darstellung wäre erheblich verzerrt und u.U. irreführend.

Wie beim herkömmlichen BSPM ist es ist wichtig, eine Korrektur der Basislinie (vertikale Anhebung) für alle Herzschlagsignale durchzuführen. Das ist häufig keine leichte Aufgabe und - wenn falsch durchgeführt - entstehen irreführende Ergebnisse der graphischen Darstellung und z.B. T-Wellen-Endpunkt-Detektion.

Die Frage reduziert sich darauf, den Zeitpunkt zu finden, an dem das EKG elektrisch stumm ist. Tatsächlich, wenn man die EKG-Signale mit hoher Amplituden-Auflösung beobachtet und Signale von räumlich verschiedenen Regionen übereinander legt, wird man feststellen, dass das EKG niemals wirklich "stumm" ist. Das ist vor allem der Fall für hohe Herzschlagraten, bei denen die T-Welle und/oder U-Welle mit der P-Welle des folgenden Herzschlags verschmelzen. Schon das Beispiel in Figur 7 zeigt das Problem, obwohl es einfach wäre, noch problematischere Fälle aufzuzeigen.

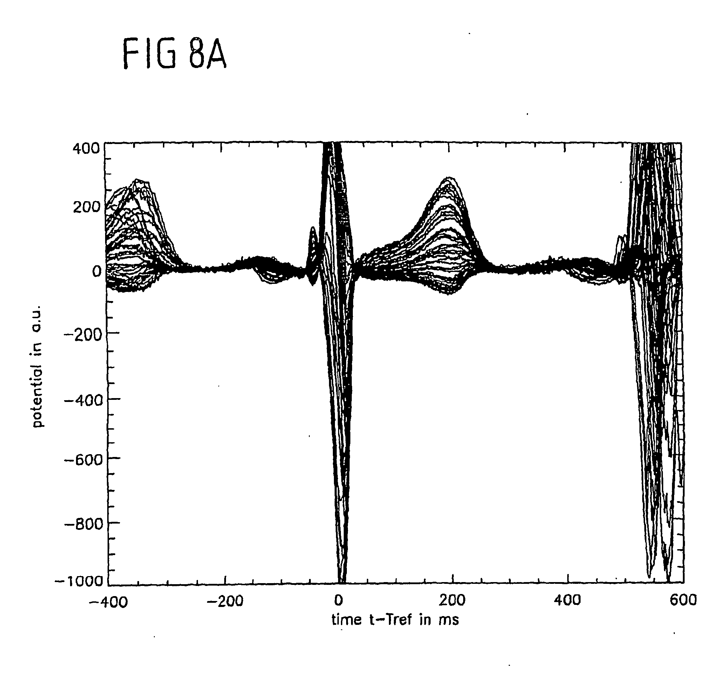

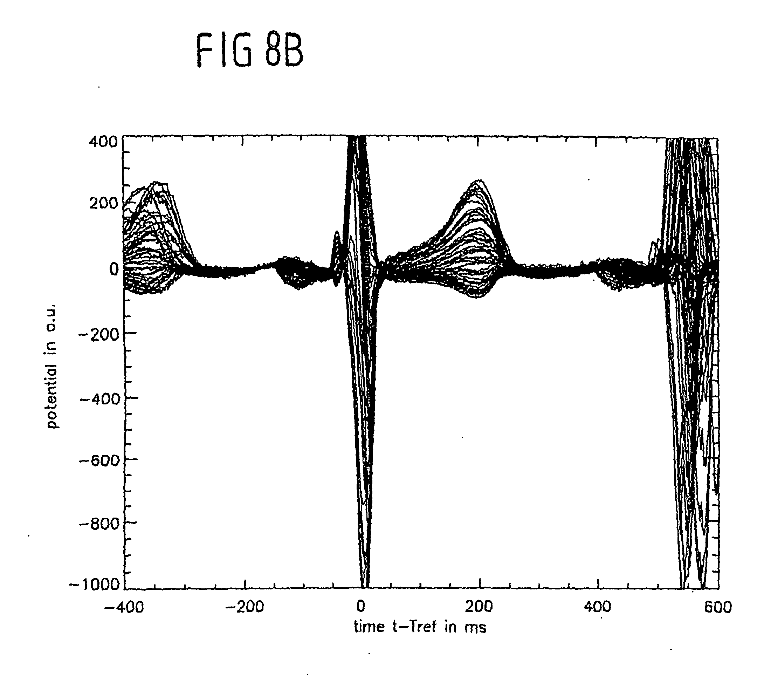

Figuren 8a und 8b zeigen sich unterscheidende Ergebnisse für die Einstellung der Basislinie für die Zeiten $T_a$ und $T_b$, gekennzeichnet in Figur 7. Auf den ersten Blick würde man den Zeitpunkt $T_b$ als die naheliegendste Wahl heranziehen, da er am weitesten hinter der Repolarisationsphase des vorangegangenen Herzschlags und gerade vor der atrialen Erregung liegt. Zu dieser Zeit sollte die gesamte Erregung elektrisch am ruhigsten sein.

Wenn zu diesem Zeitpunkt eine räumliche Verteilung von elektrischen Potentialunterschieden immer noch existiert, dann würde diese "Korrektur" der Basislinie eine Verzerrung aller individuellen Kanal-Signale verursachen und könnte Abbildungsmuster "kreieren" , z.B. für die Periode zwischen P-Welle und Beginn Q-Zacke, die in Wirklichkeit nicht existieren. Ein Vergleich von Figur 8a mit Figur 8b zeigt gerade einen solchen Effekt. Die Ausbreitung der Signalamplituden während dieser Periode zwischen atrialer und mycardialer Erregung ist in Figur 8b stärker als in Figur 8a.

Ein weiterer Grund zur Vorsicht kann von dem zusätzlichen Signal in Figur 7 (die einzelne Linie über dem Großteil der Signale) abgeleitet werden. Dieses Signal ist die Varianz aller Steigungen der darunter liegenden Signale. In anderen Worten: Wenn die Rate der Signalamplitudenänderung für alle Elektroden-Positionen die Gleiche wäre, dann wäre die Varianz gleich null. Folglich gibt ein Minimum dieses zusätzlichen Signals die Zeit minimaler elektrischer Aktivität an.

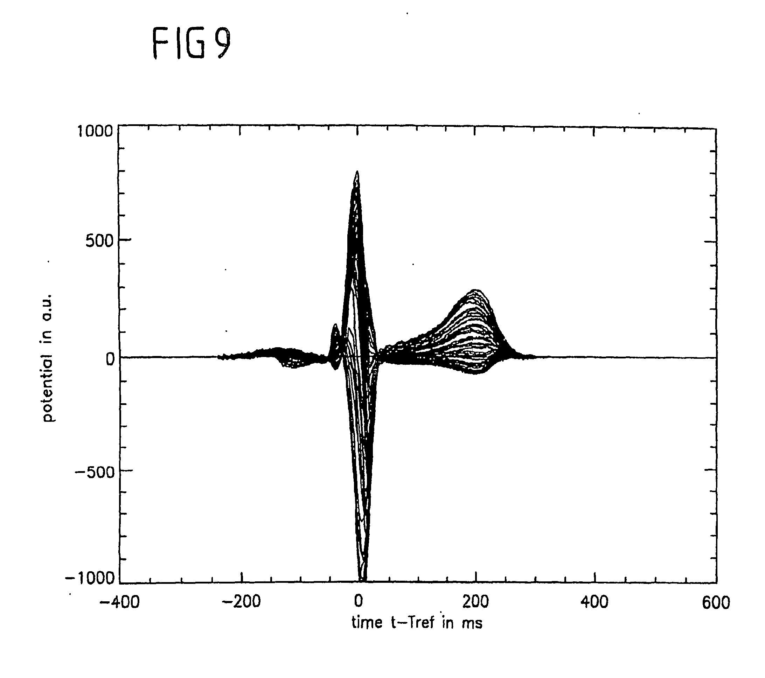

Obwohl der Zeitpunkt $T_a$ möglicherweise nicht die ideale Wahl für die Korrektur der Basislinie ist, scheint er dennoch passender zu sein als $T_b$ und wurde gewählt um Figur 9 zusammenzustellen, den finalen "charakteristischen Herzschlag", der die synchronisierten Signale aller Thoraxableitungen enthält.

**Zusammenstellung von Pseudo-BSPMs**

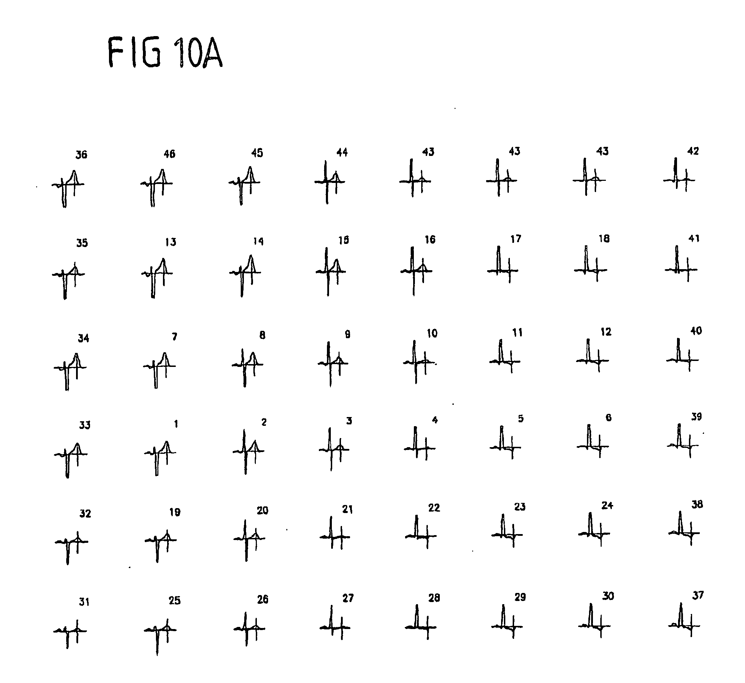

Aus dem charakteristischen Herzschlag der Figur 9 könnte wie unten beschrieben eine Vielzahl von unterschiedlichen BSPM-Plots generiert werden. Figur 10a zeigt die individuellen EKG-Signale für jede Elektroden-Position, dargestellt in einem rechtwinkligen Koordinatensystem, in Übereinstimmung mit Figur 1b. Der Cursor markiert den Zeitpunkt von $|T|_{max}$.

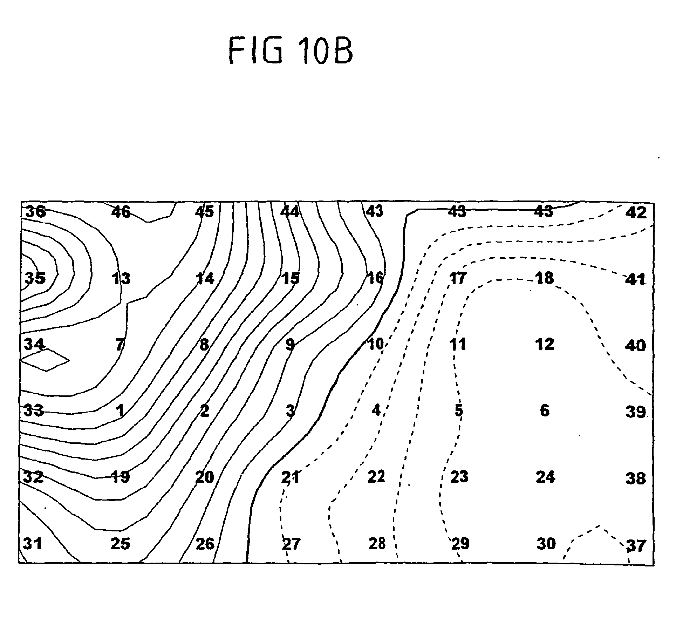

Legt man diese Amplitudenwerte zugrunde, kann ein BSPM-Plot in Höhenliniendarstellung erstellt werden, wie in Fig. 10b.

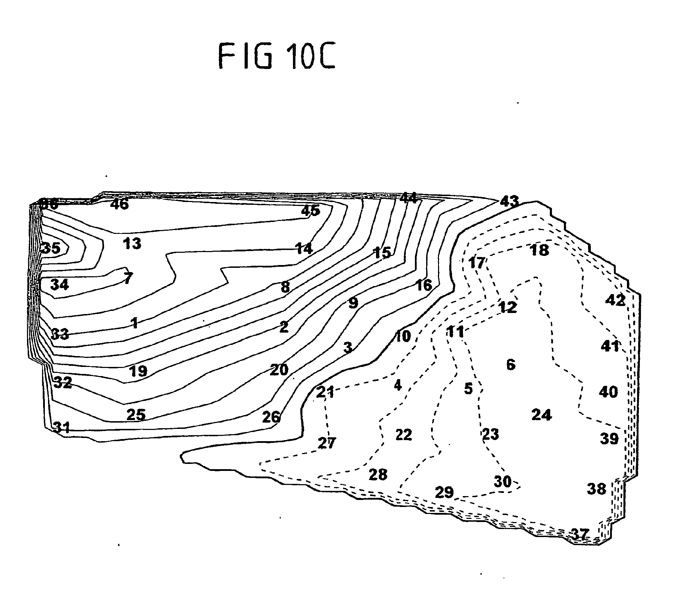

In Figur 10b und Figur 10c sind zwei unterschiedliche graphische Darstellungen für denselben Zeitpunkt der maximalen T-Wellen-Amplitude in Figur 9 gezeigt. In Figur 10b sind die Gitterpunkte in einem Quadratgittermuster arrangiert. Die Ziffernbezeichnung der korrespondierenden Elektrodenpositionen, dargestellt in Figur 1b, sind darüber gelegt. Somit ist die Potentialfeldverteilung mit Absicht verzerrt, um eine rechtwinklige Standarddarstellung zu erhalten, die mathematisch bzw. grafisch einfacher zu handhaben ist. In Figur 10c wurden die Koordinaten der Gitterpunkte geometrisch direkt in Bezug auf Figur 1b festgelegt, so dass sie weniger Verzerrung und eine realistischere Potentialfeldverteilung darstellen. Auch wenn Figur 1b eine Art 2D-Projektion einer 3-D-Realität ist, beinhaltet sie aber noch nennenswerte Verzerrung. Zusätzlich werden durch die Triangulation und Extrapolationsverarbeitung notwendigerweise signifikante Artefakte vor allem an den Rändern hinzugefügt. Somit kann der Display-Typ in Figur 10b bevorzugt werden.

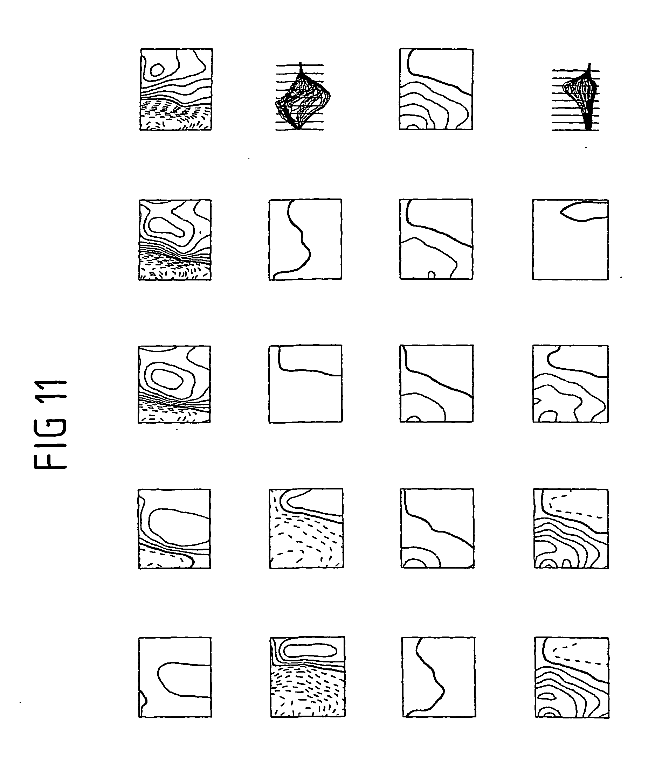

In Figur 11 sind zwei Sequenzen von graphischen Darstellungen gezeigt, die die Entwicklung der Depolarisations- und Repolarisationsphase visualisieren, d.h. neun gleichlange Zeitschritte gezeigt durch die Cursors. Ein noch eindrucksvolleres Bild der Dynamiken entsteht durch Kombination solcher Darstellungen (mit einer höheren Messrate) zu einem Video-Clip.

Letztlich ist in auch eine so genannte QRST-Integral-Darstellung möglich, bei der die Amplitudenwerte $s_n$ der Oberflächenfunktion an den Gitterpunkten die individuellen Integrale

$$s_n = \int\limits_{T_{onset}}^{T_{end}} |a_n(t)| dt \qquad\qquad (3)$$

der individuellen charakteristischen Herzschlagsignale der korrespondierenden Elektrodenpositionen n sind.

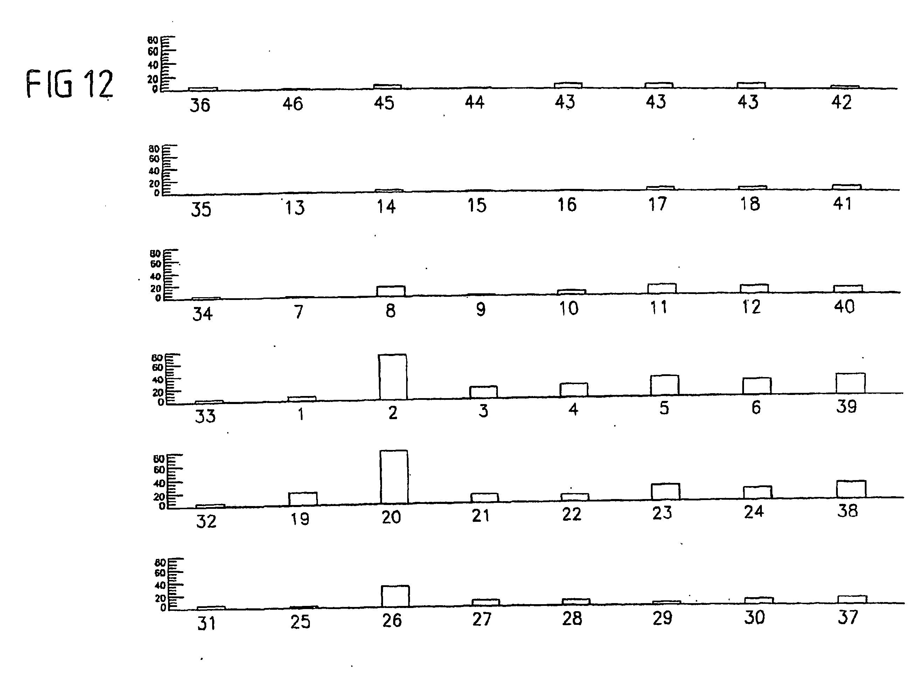

Fig. 12 zeigt das Ergebnis der räumlichen Häufigkeitsverteilung der Maxima oder Minima von 300 QRST-Integral-Plots.

Somit sind die meisten Display-Typen, die von dem traditionellen BSPM bekannt sind, bei der hier vorgestellten Methode möglich.

Wie bereits oben erwähnt, kann bei einer Ausführungsform des erfindungsgemäßen Systems oder des erfindungsgemäßen Verfahrens mindestens eine Validitätskenngröße ermittelt werden. Diese dient der Überprüfung, ob das Herzschlagmuster während aller acht Messphasen konstant ist. Dazu wird mittels des Datenverarbeitungssystems 30 aus den mit dem zweiten Messmittel 20 erfassten Extremitätenableitungen die Varianz der RR-Abstände und / oder der QT-Zeiten aller Herzschläge ermittelt. Übersteigt diese Varianz einen bestimmten Schwellenwert (z.B. 5% des zugehörigen Mittelwertes), sollte das Ergebnis der Untersuchung verworfen werden. Alternativ kann die Validitätskenngröße auch anhand eines Vergleiches der Mittelwerte der RR-Abstände und/oder der QT-Zeiten für eine Messphase mit dem zugehörigen Mittelwert für alle Messphasen bestimmt werden. Weicht einer der Mittelwerte der individuellen Phasen um mehr als einen bestimmten Schwellwert (z.B. 5%) vom zugehörigen globalen Mittelwert ab, so sollte die Messung verworfen werden. Prinzipiell könnte eine Validitätsgröße allein oder auch zusätzlich mittels der Thoraxableitungen vorgenommen werden, wobei zu berücksichtigen ist, dass durch das Versetzen der Elektroden eine Veränderung des Signalmusters entstehen könnte.

Mit dem erfindungsgemäßen Verfahren und System ist es möglich, sich der Darstellung eines üblichen body surface potential mappings (BSPM) zu einem hohen Grad anzunähern. Diese Ergebnisse können mit jedem kommerziellen Standard-12-Kanal-EKG-Gerät mit digitalem Datenoutput erzielt werden. Die Zusammenstellung eines so genannten charakteristischen Herzschlags und grafische Ausgabe von Pseudo-BSPM ist mit nur wenigen Berechnungen möglich. Die meisten digitalen 12-Kanal-EKG-Geräte erlauben die Durchführung dieser Methode einfach durch Updaten der Software durch wenige algorithmische Module.

Das Pseudo-BSPM kann zum Standard-12-Kanal-EKG klinisch relevante Informationen beitragen. Es sollte beachtet werden, dass alle 12-Kanal-EKG-Daten vollständig und automatisch in der Aufzeichnung des Pseudo-BSPM enthalten sind, vgl. Phase 1 in Figur 3. Das Pseudo-BSPM ist kommerziell attraktiv, da es keine nennenswerten zusätzlichen Kosten zum 12-Kanal-EKG-System verursacht. Wenn das Pseudo-BSPM populär wird, sollte es erneutes Interesse am echten BSPM wecken und dabei helfen, seine Verbreitung als auch seine Anwendung zu streuen.

**Figurenbeschreibung**

Figur 1
Elektrodenpositionen für a) die Brustelektroden eines konventionellen 12-Kanal-EKGs und b) für das erfindungsgemäße System. Positionen 37 bis 42 sind zu den Positionen 30, 24, 6, 12, 18, 43 benachbart, aber lateral am Thorax.

Figur 2
Schematische Darstellung der Thorax- und Extremitätenableitungen für das erfindungsgemäße System.

Figur 3

EKG-Signale, die während der ersten drei Phasen einer Aufzeichnungs-Sitzung gewonnen werden. Die Signale der Extremitätenelektroden (I, II, III, aVR, aVL, aVF) sind kontinuierlich aufgezeichnet worden, während die Brustelektroden zwischen verschiedenen Phasen wie in Figur 1b dargestellt nach oben verschoben werden. Die individuellen Signalspuren sind durch die entsprechenden Elektrodenpositionen gekennzeichnet. Die vertikalen Cursorlinien markieren Referenzzeitpunkte $t_1$, $t_2$, $t_3$.

Figur 4

Darstellung des Effekts von verschiedenen Definitionen der Referenzzeitpunkte auf die resultierenden BSPM-Plots: a) reale zeitliche Ausrichtung der QRS-Signale von verschiedenen Elektrodenpositionen. Die gestrichelten und gepunkteten Cursorlinien dienen der Ausrichtung. b) zugehöriger BSPM-Plot. Dabei stellen die dicke durchgezogene Linie die Nulllinie dar. Die durchgezogenen Linien sind positive Potentialwert, die gestrichelten Linien sind negative Potentiallinien. c) zeitliche Ausrichtung derselben Signale an Hand des Signal-Peaks als Anpassungskriterium. d) resultierender BSPM-Plot, wobei für die Äquipotentiallinien die gleiche Konvention wie für Fig. 4b gilt..

Figur 5

Bestimmung des Referenzzeitpunkts $T_{ref}$. Zum Vergleich ist oben das Signal von Kanal "I" hinzugefügt (nach oben angehoben um 600 a.u.). Die untere Spur ist das Ergebnis der Anwendung von Gleichung (1) für alle Extremitäten-Signale. $T_{ref}$ ist in den Grenzen $T_0$ und $T_e$ durch die Gleichung (2) festgelegt.

Figur 6

Bestimmung eines charakteristischen einzelnen Herzschlagsignals für eine Elektroden-Position. a) Ausrichtung und Übereinanderlegen von allen Herzschlagsignalen innerhalb einer Phase für eine Elektroden-Position. b) Median des Signals extrahiert aus a).

Figur 7

Superposition der Median-Signale von allen 48 Elektroden-Positionen von Figur 1b). Hinzugefügt ist eine zusätzliche Spur, die aus der Varianz aller Steigungen der darunter liegenden Signale gewonnen wurde. Die Cursor markieren mehrere unterschiedliche Zeitpunkte, die für eine Basislinienkorrektur in Frage kommen

Figur 8

Darstellungen mit korrigierter Basislinie : Korrektur a) in Bezug auf den Zeitpunkt $T_a$ in Figur 7 und b) in Bezug auf $T_b$.

Figur 9

Endgültige Darstellung eines charakteristischen Herzschlags.

Figur 10

Herstellung eines Pseudo-BSPM aus dem charakteristischen Herzschlag in Figur 9 zum Zeitpunkt des T-Wellen-Maximums: a) EKG-Signale von allen Kanälen geometrisch arrangiert entsprechend dem Gitter in Figur 10b. Der Cursor markiert den Zeitpunkt $T_{max}$. Die Signalamplituden dieses Zeitpunkts sind in Fig. 10b als Potentiallinien dargestellt. In c) ist das Gitter nicht rechtwinklig sondern entsprechend Figur 1b arrangiert. Die Ziffernbezeichnung der jeweiligen Elektroden-Positionen sind an den Gitterpunkten markiert. Für Potentiallinien der Fig. 10b und 10c gilt die gleich Konvention, wie für Fig. 4b.

Figur 11

Darstellung der räumlich-zeitlichen Entwicklung der Repolarisationsphase des charakteristischen Herzschlags von Figur 9 durch eine Sequenz von BSPM-Plots (dargestellt mit Potentiallinien gemäß der Konvention der Fig. 4b) für äquidistante Zeitpunkte während des QRS-Komplexes (oben) und der T-Welle (unten), markiert durch die Cursor in den jeweiligen EKGs.

Figur 12

Beispiel für die Verteilung von BSPM-Eigenschaften in Bezug auf die Elektroden-Positionen für 300 Patienten: für jede Elektroden-Position (Elektrodenziffer an der X-Achse angegeben) ist die Höhe des zugewiesenen Balkens ein Maß für die Anzahl der Patienten deren Integral-QRS-Abbildungs-Maxima oder -Minima auf die entsprechende Elektroden-Position gefallen sind. Nur 28 Prozent der Maxima oder Minima stimmen mit den konventionellen Brustelektroden $V_1$ bis $V_6$ überein.

**Patentansprüche**

1. EKG-System zur großflächigen Aufnahme von EKG-Signalen, umfassend

   ein erstes Messmittel (10) zur Erzeugung eines ersten Messdatensatzes enthaltend mindestens eine Ableitung der Herzströme, wobei mindestens ein Ableitungsort des ersten Messmittels (10) während der Aufnahme der großflächigen EKG-Signale variabel ist, und

   ein zweites Messmittel (20) zur zeitgleichen Erzeugung eines zweiten Messdatensatzes enthaltend mindestens eine Ableitung der Herzströme, wobei der Ableitungsort des zweiten Messmittels (20) während der Aufnahme der großflächigen EKG-Signale zum Erhalten kontinuierlicher Messergebnisse räumlich unveränderlich ist, **gekennzeichnet durch**

   ein Datenverarbeitungssystem (30) mit einem Mittel zur Synchronisation mindestens zweier zeitlich versetzt ermittelter Signale des ersten Messdatensatzes mit mindestens einem kontinuierlich ermittelten Signal des zweiten Messdatensatzes.

2. EKG-System nach Anspruch 1, **dadurch gekennzeichnet, dass** der erste Messdatensatz Messungen von Herzströmen enthält, die an Thorax-Ableitungen ($V_1$ - $V_6$) gewonnen sind.

3. EKG-System nach Anspruch 1 oder 2, **dadurch gekennzeichnet, dass** der erste Messdatensatz Messungen der Herzströme aus einer zeitlichen Folge von Thorax-Ableitungen ($V_1$ - $V_6$) an unterschiedlichen Thoraxpositionen enthält.

4. EKG-System nach mindestens einem der vorhergehenden Ansprüche, **dadurch gekennzeichnet, dass** der zweite Messdatensatz mindestens eine Messung der Herzströme einer Extremitätenableitung (I, II, III, aVR, aVL, aVF) enthält.

5. EKG-System nach Anspruch 4, **dadurch gekennzeichnet, dass** der zweite Messdatensatz Signale der Herzströme aller Extremitätenableitungen (I, II, III, aVR, aVL, aVF) enthält.

6. EKG-System nach mindestens einem der vorhergehenden Ansprüche, **dadurch gekennzeichnet, dass** die Synchronisation anhand mindestens eines markanten Signalmusters des zweiten Messdatensatzes erfolgt.

7. EKG-System nach Anspruch 6, **dadurch gekennzeichnet, dass** das Mittel zur Synchronisation das Signal einer R-Zacke im zweiten Messdatensatz zur Synchronisation verwendet.

8. EKG-System nach Anspruch 7, **dadurch gekennzeichnet, dass** das Mittel zur Synchronisation das Signal der Steigung der R-Zacke im zweiten Messdatensatz zur Synchronisation verwendet.

9. EKG-System nach mindestens einem der Ansprüche 6 bis 8, **dadurch gekennzeichnet, dass** das Mittel zur Synchronisierung markante Signalmaker mehrerer gemessener EKG-Kanäle verwendet.

10. EKG-System nach mindestens einem der vorhergehenden Ansprüche, **gekennzeichnet durch** einen Filter, ein Mittel zur Mittelung und/oder zur Ermittlung des Medians für Signale des ersten Messdatensatzes und/oder des zweiten Messdatensatzes.

11. EKG-System nach mindestens einem der vorhergehenden Ansprüche, **gekennzeichnet durch** ein Mittel zur Korrektur der Basislinie individueller Herzströme.

12. EKG-System nach mindestens einem der vorhergehenden Ansprüche, **dadurch gekennzeichnet, dass** mit dem Datenverarbeitungssystem (30) für jeden beliebigen Zeitpunkt einer Messung relativ zu einer mittels eines Signals des zweiten Messdatensatzes gewonnenen Zeitreferenz aus den Amplitudenwerten aller Thorax-Ableitungen automatisch eine graphische Darstellung der momentanen Potentialverteilung ermittelbar ist.

13. EKG-System nach Anspruch 12, **dadurch gekennzeichnet, dass** die graphische Darstellung eine QRST-Integral-Map Darstellung ist.

14. EKG-System nach mindestens einem der vorhergehenden Ansprüche, **dadurch gekennzeichnet, dass** das erste Messmittel (10) und/oder das zweite Messmittel (20) in einer am menschlichen Körper tragbaren Vorrichtung, insbesondere einer Weste angeordnet sind.

15. EKG-System nach mindestens einem der vorhergehenden Ansprüche, **dadurch gekennzeichnet, dass** mittels des Datenverarbeitungssystems (30) eine Varianz von Messergebnissen als Validitätskenngröße ermittelbar ist.

16. EKG-System nach Anspruch 15, **dadurch gekennzeichnet, dass** die Varianz der Messergebnisse anhand eines Maßes bestimmter EKG-Potentialhöhen, insbesondere von RR-Abständen, QT-Zeiten und / oder eines Vergleiches eines Mittelwertes eines Maßes für eine EKG-Potentialhöhe einer Messphase mit dem Mittelwert für Maße von EKG-Potentialhöhen aller Messphasen ermittelbar ist.

17. Verfahren zur großflächigen Aufnahme von EKG-Signalen, umfassend
eine Aufnahme mindestens einer ersten Messung der Herzströme mit einem ersten Messmittel (10), wobei mindestens ein Ableitungsort eines ersten Messmittels (10) während der Aufnahme der großflächigen EKG-Signale verändert wird,
eine zeitgleiche Aufnahme mindestens einer zweiten Messung der Herzströme mit einem zweiten Messmittel (20), wobei der Ableitungsort des zweiten Messmittels (20) während der Aufnahme der großflächigen EKG-Signale zur kontinuierlichen Messung räumlich unveränderlich ist,
das erste und zweite Messmittel (10, 20) einen ersten Messdatensatz und einen zweiten Messdatensatz erzeugen, und sofort oder zu einem späteren Zeitpunkt mindestens zwei zeitlich versetzt ermittelte Signale der Herzströme des ersten Messdatensatzes mit mindestens einem kontinuierlich ermittelten Signal des zweiten Messdatensatzes der Herzströme in einem Datenverarbeitungssystem (30) automatisch synchronisiert werden.

18. Verfahren nach Anspruch 17, **dadurch gekennzeichnet, dass** insbesondere zur Simulation eines Body-Surface-Potential-Mappings mindestens zwei erste Ableitungen durch einen Interkostalabstand getrennt am Thorax gewonnen werden.

**Claims**

1. An ECG system for large-surface recording of ECG signals, comprising
a first measuring means (10) for generating a first measured data record including at least one reading of the cardiac currents, at least one lead site of the first measuring means (10) being variable during the recording of the large-surface ECG signals, and
a second measuring means (20) for simultaneously generating a second measured data record including at least one reading of the cardiac currents, the lead site of the second measuring means (20) being spatially invariable during the recording of the large-surface ECG signals in order to obtain continuous measurement results,
**characterized by**
a data processing system (30) having a means for synchronizing at least two signals, determined in a temporally offset fashion, of the first measured data record with at least one continuously detected signal of the second measured data record.

2. The ECG system as claimed in claim 1, **characterized in that** the first measured data record includes measurements of cardiac currents that have been obtained at thorax leads ($V_1$ - $V_6$).

3. The ECG system as claimed in claim 1 or 2, **characterized in that** the first measured data record includes measurements of the cardiac currents from a temporal sequence of thorax leads ($V_1$ - $V_6$) at different thorax positions.

4. The ECG system as claimed in at least one of the preceding claims, **characterized in that** the second measured data record includes at least one measurement of the cardiac currents of an extremity lead (I, II, III, aVR, aVL, aVF).

5. The ECG system as claimed in claim 4, **characterized in that** the second measured data record includes signals of the cardiac currents of all the extremity leads (I, II, III, aVR, aVL, aVF).

6. The ECG system as claimed in at least one of the preceding claims, **characterized in that** the synchronization is performed with the aid of at least one prominent signal pattern of the second measured data record.

7. The ECG system as claimed in claim 6, **characterized in that** the means for synchronizing uses the signal of an R wave in the second measured data record for the purpose of synchronization.

8. The ECG system as claimed in claim 7, **characterized in that** the means for synchronizing uses the signal of the rise in the R wave in the second measured data record for the purpose of synchronization.

9. The ECG system as claimed in at least one of claims 6 to 8, **characterized in that** the means for synchronizing uses prominent signal markers of a number of measured ECG channels.

10. The ECG system as claimed in at least one of the preceding claims, **characterized by** a filter, a means for averaging and/or for determining the median for signals of the first measured data record and/or of the second measured data record.

11. The ECG system as claimed in at least one of the preceding claims, **characterized by** a means for correcting the baseline of individual cardiac currents.

12. The ECG system as claimed in at least one of the preceding claims, **characterized in that** the data processing system (30) can use the amplitude values of all the thorax readings to determine a graphic display of the instantaneous potential distribution automatically for any desired instant of a measurement relative to a time reference obtained by means of a signal of the second measured data record.

13. The ECG system as claimed in claim 12, **characterized in that** the graphic display is a QRST integral map display.

14. The ECG system as claimed in at least one of the preceding claims, **characterized in that** the first measuring means (10) and/or the second measuring means (20) are/is arranged in a contrivance, in particular a vest, that can be worn on the human body.

15. The ECG system as claimed in at least one of the preceding claims, **characterized in that** a variance of measurement results can be ascertained as a validity characteristic by means of the data processing system (30).

16. The ECG system as claimed in claim 15, **characterized in that** the variance of the measurement results can be ascertained with the aid of a measure of specific ECG potential levels, in particular R-R intervals, QT times and/or of a comparison of a mean value of a measure of an ECG potential level of one measurement phase with the mean value for measures of ECG potential levels of all the measurement phases.

17. A method for large-surface recording of ECG signals, comprising
a recording at least one first measurement of the cardiac currents with the aid of a first measuring means (10), at least one lead site of a first measuring means (10) being varied during recording of the large-surface ECG signals, simultaneously recording at least one second measurement of the cardiac currents with the aid of a second measuring means (20), the lead site of the second measuring means (20) being spatially invariable during recording of the large-surface ECG signals for the purpose of continuous measurement,
the first and second measuring means (10, 20) generating a first measured data record and a second measured data record,
and immediately or at a later instant, at least two signals, determined in a temporally offset fashion, of the cardiac currents of the first measured data record being automatically synchronized in a data processing system (30) with at least one continuously determined signal of the second measured data record of the cardiac currents.

18. The method as claimed in claim 17, **characterized in that** at least two first readings are obtained on the thorax in a fashion separated by an intercostal spacing, in particular for the purpose of simulating a body surface potential mapping.

**Revendications**

1. Système ECG pour l'enregistrement en grand format de signaux ECG, comprenant :

   un premier moyen de mesure (10) pour générer un premier jeu de données de mesure contenant au moins une dérivation des flux cardiaques, au moins un point de dérivation du premier moyen de mesure (10) étant variable pendant l'enregistrement des signaux ECG en grand format, et
   un deuxième moyen de mesure (20) pour la génération simultanée d'un deuxième jeu de données de mesure contenant au moins une dérivation des flux cardiaques, le point de dérivation du deuxième moyen de mesure (20) ne variant pas dans l'espace pendant l'enregistrement des signaux ECG en grand format afin de recevoir des résultats de mesure continus,

**caractérisé par** un système de traitement de données (30) avec un moyen pour la synchronisation d'au moins deux signaux détectés de manière décalée dans le temps du premier jeu de données de mesure avec au moins un signal détecté de manière continue du deuxième jeu de données de mesure.

2.  Système ECG selon la revendication 1, **caractérisé en ce que** le premier jeu de données de mesure contient des mesures de flux cardiaques ayant été obtenues au niveau de dérivations du thorax (V1 - V6).

3.  Système ECG selon la revendication 1 ou 2, **caractérisé en ce que** le premier jeu de données de mesure contient des mesures des flux cardiaques issues d'une suite chronologique de dérivations du thorax (V1 - V6) au niveau de différentes positions du thorax.

4.  Système ECG selon au moins l'une quelconque des revendications précédentes, **caractérisé en ce que** le deuxième jeu de données de mesure contient au moins une mesure des flux cardiaques d'une dérivation d'extrémité (I, II, III, aVR, aVL, aVF).

5.  Système ECG selon la revendication 4, **caractérisé en ce que** le deuxième jeu de données de mesure contient des signaux des flux cardiaques de toutes les dérivations d'extrémités (I, II, III, aVR, aVL, aVF).

6.  Système ECG selon au moins l'une quelconque des revendications précédentes, **caractérisé en ce que** la synchronisation est réalisée à l'aide d'au moins un motif de signal caractéristique du deuxième jeu de données de mesure.

7.  Système ECG selon la revendication 6, **caractérisé en ce que** le moyen pour la synchronisation utilise le signal d'une onde R dans le deuxième jeu de données de mesure pour la synchronisation.

8.  Système ECG selon la revendication 7, **caractérisé en ce que** le moyen pour la synchronisation utilise le signal du flanc ascendant de l'onde R dans le deuxième jeu de données de mesure pour la synchronisation.

9.  Système ECG selon au moins l'une quelconque au moins des revendications 6 à 8, **caractérisé en ce que** le moyen pour la synchronisation utilise des générations de signaux caractéristiques de plusieurs canaux ECG mesurés.

10.  Système ECG selon au moins l'une quelconque au moins des revendications précédentes, **caractérisé par** un filtre, un moyen pour calculer la moyenne et/ou déterminer la médiane pour des signaux du premier jeu de données de mesure et/ou du deuxième jeu de données de mesure.

11.  Système ECG selon au moins l'une quelconque au moins des revendications précédentes, **caractérisé par** un moyen pour corriger la ligne de base de flux cardiaques individuels.

12.  Système ECG selon l'une quelconque au moins des revendications précédentes, **caractérisé en ce qu'**avec le système de traitement de données (30) il est possible de déterminer automatiquement, pour chaque moment au choix d'une mesure par rapport à une référence temporelle acquise à l'aide d'un signal du deuxième jeu de données de mesure à partir des valeurs d'amplitude de toutes les dérivations de thorax, une représentation graphique de la répartition momentanée du potentiel.

13.  Système ECG selon la revendication 12, **caractérisé en ce que** la représentation graphique est une représentation QRST-Integral-Map.

14.  Système ECG selon au moins l'une quelconque au moins des revendications précédentes, **caractérisé en ce que** le premier moyen de mesure (10) et/ou le deuxième moyen de mesure (20) sont disposés dans un dispositif pouvant être porté par un corps humain, en particulier une veste.

15.  Système ECG selon au moins l'une quelconque au moins des revendications précédentes, **caractérisé en ce qu'**à l'aide du système de traitement de données (30), il est possible de déterminer une variation des résultats de mesure en tant que grandeur nominale de validité.

16.  Système ECG selon la revendication 15, **caractérisé en ce que** la variation des résultats de mesure peut être déterminée à l'aide d'une grandeur de niveaux de potentiel d'ECG déterminés, en particulier des écarts RR, des temps QT et/ou d'une comparaison entre une valeur médiane d'une grandeur pour un niveau de potentiel d'ECG d'une phase de mesure et une valeur médiane pour des grandeurs de niveaux de potentiels d'ECG de toutes les phases de mesure.

17. Procédé pour l'enregistrement en grand format de signaux ECG, comprenant un enregistrement d'au moins une première mesure des flux cardiaques avec un premier moyen de mesure (10), au moins un point de dérivation d'un premier moyen de mesure (10) étant modifié pendant l'enregistrement des signaux ECG en grand format, un enregistrement simultané d'au moins une deuxième mesure des flux cardiaques avec un deuxième moyen de mesure (20), le point de dérivation du deuxième moyen de mesure (20) ne variant pas dans l'espace pendant l'enregistrement des signaux ECG en grand format pour la mesure continue, le premier et le deuxième moyens de mesure (10, 20) générant un premier jeu de données de mesure et un deuxième jeu de données de mesure, et immédiatement ou à un moment plus tard, au moins deux signaux détectés de manière décalée dans le temps des flux cardiaques du premier jeu de données de mesure étant automatiquement synchronisés avec au moins un signal déterminé en continu du deuxième jeu de données de mesure des flux cardiaques dans un système de traitement de données (30).

18. Procédé selon la revendication 17, **caractérisé en ce que**, en particulier pour la simulation d'un Body-Surface-Potential-Mapping, au moins deux premières dérivations grâce à un écart intercostal sont acquises séparément au niveau du thorax.

# FIG 1A

# FIG 1B

# FIG 2

V1  V2  V5  V6
V3
V4

30

20

I

II   III

R           L           F

aVR         aVL         aVF

FIG 3

FIG 4A

FIG 4B

EP 1 653 851 B1

FIG 4D

FIG 4C

FIG 5

EP 1 653 851 B1

FIG 6A

FIG 7

FIG 8A

EP 1 653 851 B1

## FIG 8B

FIG 9

EP 1 653 851 B1

FIG 10A

## FIG 10B

EP 1 653 851 B1

FIG 10C

30

FIG 11

FIG 12

EP 1 653 851 B1

# EP 1 653 851 B1

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5377687 A

**In der Beschreibung aufgeführte Nicht-Patentliteratur**

- **Herausgeber P.W.Macfarlane ; T.D. Veitch Lawrie.** Comprehensive Elektrocardiography - Theory and Practice in Health and Desease. Pergamon Press, 1989, vol. 1
- Body Surface Potential Mapping. **N.C. Flower ; L.G. Horan.** Cardiac Electrophysiology - From Cell to Bedside. W.B. Saunders, 2000
- **A.P. Michaelides et al.** Improved detection of coronary artery disease by exercise electrocardiography with the use of right precordial leads. *N. Eng. J. Med.,* 1999, vol. 340, 5
- **von Bruno Taccardi.** Distribution of Heart Potentials on the Thoracic Surface of Normal Human Subjects. *Circulation Research,* April 1963, vol. XII

- Body Surface Potential Mapping. **N.C. Flowers ; L.G. Horan.** Cardiac Electrophysiology: From Cell to Bedside. WB Saunders, 1995, 1049-1067
- **N.C. Flower ; L.G. Horan.** Body Surface Potential Mapping. *Cardiac Electrophysiology - From Cell to Bedside,* 2000
- Comprehensive Electrocardiology-Theory and Practice in Health and Disease. **Herausgeber P.W.Macfarlane ; T.D. Veitch Lawrie.** Comprehensive Elektrocardiography - Theory and Practice in Health and Desease. Pergamon Press, 1989, vol. 1